# EUROPEAN PATENT SPECIFICATION

(11) **EP 2 117 553 B1**
(45) Date of publication and mention of the grant of the patent: **31.08.2011**
(21) Application number: 08729826.1
(22) Date of filing: 14.02.2008
(51) Int. Cl.: A61K 31/55, A61P 11/00, A61P 27/16

(54) **COMPOSITIONS FOR TREATING, REDUCING, AMELIORATING, OR PREVENTING INFECTIONS OF THE EAR OR UPPER RESPIRATORY TRACK**
ZUSAMMENSETZUNGEN ZUR BEHANDLUNG, REDUKTION, VERMINDERUNG ODER VERHINDERUNG VON INFEKTIONEN DES OHRS ODER DER OBEREN ATEMWEGE
COMPOSITIONS POUR LE TRAITEMENT, LA RÉDUCTION, L'AMÉLIORATION, OU LA PRÉVENTION D'INFECTIONS DE L'OREILLE OU DE LA VOIE RESPIRATOIRE SUPÉRIEURE

(30) Priority: 16.02.2007 US 675930
(43) Date of publication of application: 18.11.2009
(73) Proprietor: Bausch & Lomb Incorporated, Rochester, NY 14604-2701 (US)
(72) Inventor: VENKATESH, Srini, Pittsford, NY 14534 (US); JONASSE, Matthew, Scott, Webster, NY 14580 (US); WANG, Hongna, Fairport, NY 14450 (US)
(74) Representative: Glas, Holger
(86) International application number: PCT/US2008/053918
(87) International publication number: WO 2008/101052

(56) References cited:
- WO-A-2008/045673
- US-A- 5 385 900
- US-A1- 2002 187 193
- GOLDSTEIN ELLIE J C ET AL: "In vitro activities of ABT-492, a new fluoroquinolone, against 155 aerobic and 171 anaerobic pathogens isolated from antral sinus puncture specimens from patients with sinusitis." ANTIMICROBIAL AGENTS AND CHEMOTHERAPY, vol. 47, no. 9, September 2003 (2003-09), pages 3008-3011, XP002484124 ISSN: 0066-4804
- ZHANEL GEORGE G ET AL: "A critical review of the fluoroquinolones: Focus on respiratory tract infections" DRUGS, vol. 62, no. 1, 2002, pages 13-59, XP009101496 ISSN: 0012-6667
- KURIYAMA H ET AL: "Efficacy of the NY-198 (lomefloxacin) otic solution on experimental otitis media in guinea pigs" JAPANESE PHARMACOLOGY AND THERAPEUTICS 1990 JP, vol. 18, no. 12, 1990, pages 49-58, XP009101494 ISSN: 0386-3603
- YAGUPSKY P ET AL: "In vitro activity of novel fluoroquinolones against Streptococcus pneumoniae isolated from children with acute otitis media" CHEMOTHERAPY, vol. 47, no. 5, September 2001 (2001-09), pages 354-358, XP009101562 ISSN: 0009-3157
- BARZA M: "USE OF QUINOLONES FOR TREATMENT OF EAR AND EYE INFECTIONS" EUROPEAN JOURNAL OF CLINICAL MICROBIOLOGY AND INFECTIOUS DISEASES, vol. 10, no. 4, 1991, pages 296-303, XP009101563 ISSN: 0934-9723
- HOOGKAMP-KORSTANJE J A A: "In-vitro activities of ciprofloxacin, levofloxacin, lomefloxacin, ofloxacin, pefloxacin, sparfloxacin and trovafloxacin against Gram-positive and Gram-negative pathogens from respiratory tract infections", JOURNAL OF ANTIMICROBIAL CHEMOTHERAPY, vol. 40, no. 3, September 1997 (1997-09), pages 427-431, ISSN: 0305-7453
- HOOGKAMP-KORSTANJE J A A: "In-vitro activities of ciprofloxacin, levofloxacin, lomefloxacin, ofloxacin, pefloxacin, sparfloxacin and trovafloxacin against Gram-positive and Gram-negative pathogens from respiratory tract infections" JOURNAL OF ANTIMICROBIAL CHEMOTHERAPY, vol. 40, no. 3, September 1997 (1997-09), pages 427-431, ISSN: 0305-7453

## Description

### BACKGROUND OF THE INVENTION

The present invention relates to compositions for use in treating, reducing, ameliorating, or preventing infections of the ear and upper respiratory tract. In particular, the present invention relates to such compositions comprising quinolone carboxylic acids, derivatives thereof, and therapeutic applications using the same.

Bacterial pathogens continue to pose a serious threat to public health as indicated by a worldwide resurgence of bacterial diseases. Otitis and sinusitis are two very common infections that are difficult to treat for a number of reasons, including bacterial resistance to antibiotics. Such resistance may be attributed to prior widespread, and largely effective, therapeutic and prophylactic use of antibiotics, which, unfortunately, over time has also selected for resistant strains of various bacterial pathogens. Of particular concern to the public health has been the emergence and proliferation of bacterial strains that are resistant to multiple antibiotics in the current arsenal of antimicrobial agents. Such multiantibiotic-resistant ("MAR") bacterial strains include species of Gram-positive bacteria, such as, antibiotic-resistant strains of *Staphylococcus aureus, Enterococcus fecalis,* and *Enterococcus fecium,* which, along with antibiotic-resistant Gram-negative strains of *Escherichia coli,* constitute the most frequent etiological agents of nosocomial (hospital-acquired) diseases, such as septicemia, endocarditis, and infections of wounds and the urinary tract. *S*. *aureus* is currently the most frequent cause of nosocomial bacteremia and skin or wound infection. *Streptococcus pneumoniae* causes several serious and life-threatening diseases, including a contagious meningitis, bacteremia, and otitis media. Annual mortality from S. *pneumoniae* infection alone is estimated at between 3-5 million persons globally. More recently, clinical accounts of highly aggressive skin and tissue infections by "flesh-eating" strains of Group-A *streptococcus* bacteria, such as *Streptococcus pyogenes,* has heightened the concern and need for new or improved antibacterial agents.

Quinolones constitute a group of antibiotics that have been available since the early 1960s and have proved to be valuable antibacterial agents. Quinolone carboxylic acid derivatives having various chemical structures have been synthesized, developed, and marketed. Nalidixic acid (1,4-dihydro-1-ethyl-7-methyl-1,8-naphthyridin-4-one-3-carboxylic acid), the progenitor of the series, was used primarily as a urinary-tract antiseptic. Later development provided agents with broader activity, increased potency against selected pathogens and improved pharmacokinetic and pharmacodynamic properties.

From a medical utility viewpoint, the quinolones are classified as first-, second-, and third-generation compounds. First-generation compounds like piromidic acid (8-ethyl-5,8-dihydro-5-oxo-2-(1-pyrrolidinyl)pyrido(2,3-d)pyrimidine-6-carboxylic acid) and pipemidic acid (8-ethyl-5,8-dihydro-5-oxo-2-(1-piperazinyl)pyrido(2,3-d)pyrimidine-6-carboxylic acid) provided coverage for Gram-negative *Enterobacteriaceae.* The second-generation compounds are divided into those with enhanced but predominant Gram-negative activity, against pathogens like *Escherischia coli* and *Pseudomonas aeruginosa,* and those with balanced broad-spectrum activity (norfloxacin, pefloxacin, enoxacin, fleroxacin, lomefloxacin, ciprofloxacin, ofloxacin, rufloxacin, nadifloxacin). Norfloxacin, ofloxacin, and ciprofloxacin have, therefore, been used mainly for treatment of diseases including urinary tract infections, gastrointestinal infections, sexually transmitted diseases and the like. Third-generation antibiotics (levofloxacin, pazufloxacin, sparfloxacin, clinafloxacin, sitafloxacin, trovafloxacin, tosufloxacin, temafloxacin, grepafloxacin, balofloxacin, moxifloxacin, gatifloxacin) are those with enhanced activity against Gram-positive cocci (notably clinafloxacin, sitafloxacin, trovafloxacin for *Streptococcus pneumoniae*) and, for essentially all the third-generation quinolones, activity also against Gram-negative *Haemophilus influenzae* and *Legionella pneumophila,* and against anaerobes and atypical pathogens. Levofloxacin, moxifloxacin, and gatifloxacin have, therefore, found use for community-acquired infections such as those of the upper and lower respiratory tract infections ("RTI") like pneumonia, sinusitis and pharyngitis, and for skin and soft tissue infections ("SSI") caused by Gram-positive strains of *staphylococci, pneumococci, streptococci,* and *enterococci.*

The improvements seen in most of the third-generation antibiotics in current use are generally attributed to their uniqueness in inhibiting DNA gyrase and topoisomerase IV of the bacterial targets. Three categories of quinolone inhibition have been suggested. Type I quinolones (norfloxacin, enoxacin, fleroxacin, ciprofloxacin, lomefloxacin, trovafloxacin, grepafloxacin, ofloxacin and levofloxacin) indicate a preference for topoisomerase IV inhibition. Type II quinolones (nadifloxacin and sparfloxacin) indicate a preference for DNA gyrase inhibition. Type III quinolones to which some of the third-generation quinolones belong (e.g., gatifloxacin, pazufloxacin, moxifloxacin and clinafloxacin) display, however, a dual-targeting property, and equally influence DNA gyrase inhibition and topoisomerase IV inhibition. (M. Takei, et al., Antimicrobial Agents and Chemotherapy, Vol. 45, 3544-49 (2001)). DNA gyrase is the primary target in bacteria, and thus is explained the weaker activity in Gram-positive bacteria of the topoisomerase IV-targeting second-generation quinolones like norfloxacin, ciprofloxacin, ofloxacin, and levofloxacin. The unusual activity of nadifloxacin described in the literature, especially against Gram-positive *S*. *aureus,* now can be explained by its ability to target DNA gyrase (N. Oizumi, et al., J. Infect. Chemother., Vol. 7, 191-194 (2001)). That some third-generation quinolones are primarily capable of targeting topoisomerase IV in Gram-positive *staphylococci,* and DNA gyrase in Gram-positive *S*. *pneumoniae,* explains the advantages provided by the dual-targeting third-generation quinolones like moxifloxacin and gatifloxacin. However, because of continuing threat of new strains of antibiotic-resistant bacteria that may surface in the future, continued effort has been devoted to develop new broad-spectrum antibiotics.

A family of fluoroquinolones was recently developed, and some compounds of this family show good antimicrobial activity against a wide range of Gram-positive and Gram-negative bacteria. See U.S. Patents 5, 385,900; 5,447,926; 6,685,958; and 6,699,492.

Furthermore, the activity of clinafloxacin, gatifloxacin, gemifloxacin, grepafloxacin, levofloxacin, moxifloxacin, sitafloxacin, sparfloxacin and trovafloxacin against Gram-negative bacilli and improved Gram-positive activity (e.g. against Streptococcus pneumoniae and Staphylococcus aureus) over ciprofloxacin has been discussed in the art. Ciprofloxacin maintained the best *in vitro* activity against Pseudomonas aeruginosa. Clinafloxacin, gatifloxacin, moxifloxacin, sitafloxacin, sparfloxacin and trovafloxacin display improved activity against anaerobes (e.g. Bacteroides fragilis) versus ciprofloxacin. Clinical trials comparing the foregoing fluoroquinolones to each other or to standard therapy have demonstrated good efficacy in a variety of community-acquired respiratory infections (e.g. pneumonia, acute exacerbations of chronic bronchitis and acute sinusitis) (Zhanel et al., Drugs, vol. 62, no. 1, 13-59 (2002)).

The efficacy of lomefloxacin otic solutions on the experimental otitis media in guinea pigs was investigated in view of *S. aureus* or *P. auruginosa.* Though 1% Cefmenoxime otic solution also suppressed otitis media, its efficacy was a little inferior to 0.1% lomefloxacin in *P. aeruginosa* induced otitis media (Kuriyama et al., Japanese Pharmacology and therapeutics, vol. 18, no. 12, 49-58 (1990)).

The treatment of malignant otitis externa has been further discussed in the art. In this context, it was stated that treatment with a quinolone has the advantage over older treatments that it can be given orally and has a low rate of side-effects. The ocular penetration of the ciprofloxacin, pefloxacin, ofloxacin or norfloxacin has been studied in patients with uninfected eyes, wherein it was not determined whether the concentrations achieved will be adequate for therapeutic or prophylactic purposes (Barza, Eur. J. Clin. Microbiol. Infect. Dis., vol. 10, no. 4, 296-303 (1991).

Therefore, it is very desirable, in one aspect, to develop improved therapeutic compositions that incorporate this family of fluoroquinolones. It is also very desirable to provide novel and more effective compositions and methods for the treatment of infection of the ear and/or upper respiratory tract. In addition, it is also very desirable to provide novel compositions and methods for the treatment, reduction, or amelioration of otitis and/or sinusitis that is caused by bacteria resistance to common antibiotics.

### SUMMARY OF THE INVENTION

In general, the present invention provides pharmaceutical compositions which can be used for the treatment, reduction, amelioration, or prevention of infection of an ear or a portion of upper respiratory tract.

In one aspect, said infection is selected from the group consisting of otitis, sinusitis, nasopharyngitis, oropharyngitis, and combinations thereof.

In another aspect, such compositions comprise at least one member of a family of fluoroquinolones that have Formula I or salts thereof, 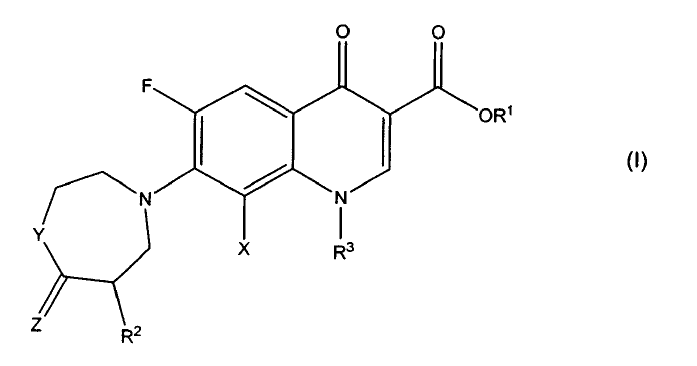 wherein R¹ is selected from the group consisting of hydrogen, unsubstituted lower alkyl groups, substituted lower alkyl groups, cycloalkyl groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, and groups that can be hydrolyzed in living bodies;
R² is selected from the group consisting of hydrogen, unsubstituted amino group, and amino groups substituted with one or two lower alkyl groups; R³ is selected from the group consisting of hydrogen, unsubstituted lower alkyl groups, substituted lower alkyl groups, cycloalkyl groups, unsubstituted lower alkoxy groups, substituted lower alkoxy groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, unsubstituted C₅-C₂₄ aryloxy groups, substituted C₅-C₂₄ aryloxy groups, unsubstituted C₅-C₂₄ heteroaryloxy groups, and substituted C₅-C₂₄ heteroaryloxy groups, X is selected from the group consisting of halogen atoms; Y is selected from the group consisting of CH₂, O, S, SO, SO₂ and NR⁴, wherein R⁴ is selected from the group consisting of hydrogen, unsubstituted lower alkyl groups, substituted lower alkyl groups, and cycloalkyl groups; and Z is selected from the group consisting of oxygen and two hydrogen atoms.

In still another aspect, a composition of the present invention comprises a single enantiomer of a compound having Formula I.

In still another aspect, a composition of the present invention comprises a member of a family of fluoroquinolones having Formula II or salts thereof, 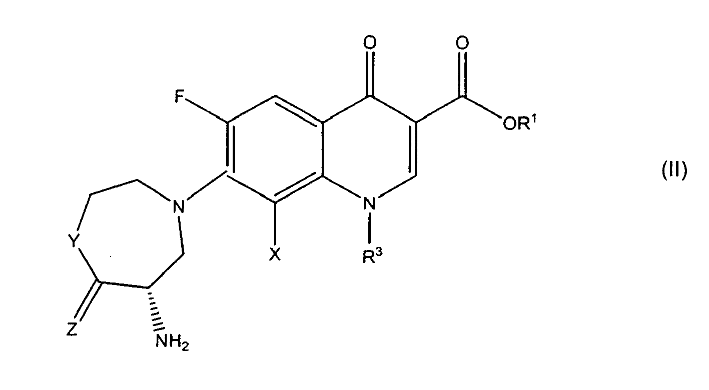 wherein R¹, R³, X, Y, and Z have the meanings as disclosed above.

The present invention provides a means for treating, reducing, ameliorating, or preventing an infection of an ear or a portion of an upper respiratory tract by administering a composition comprising a fluroquinolone having Formula I or II to a site of infection to treat, reduce, or ameliorate said infection.

In one embodiment, it is disclosed topically administering such a composition. In another embodiment it is disclosed orally administering such a composition.

In yet another aspect, said otitis is selected from the group consisting of otitis externa, otitis media, and combinations thereof.

In a further aspect, said sinusitis is selected from the group consisting of maxillary sinusitis, frontal sinusitis, ethmoid sinusitis, sphenoid sinusitis, and combinations thereof.

Other features and advantages of the present invention will become apparent from the following detailed description and claims.

### DETAILED DESCRIPTION

As used herein, the term "lower alkyl" or "lower alkyl group" means a C₁-C₁₅ linear- or branched-chain saturated aliphatic hydrocarbon monovalent group, which may be unsubstituted or substituted. The group may be partially or completely substituted with halogen atoms (F, Cl, Br, or I). Non-limiting examples of lower alkyl groups include methyl, ethyl, n-propyl, 1-methylethyl(isopropyl), n-butyl, n-pentyl, 1,1-dimethylethyl (t-butyl), and the like. It may be abbreviated as "Alk".

As used herein, the term "lower alkoxy" or "lower alkoxy group" means a C₁-C₁₅ linear- or branched-chain saturated aliphatic alkoxy monovalent group, which may be unsubstituted or substituted. The group may be partially or completely substituted with halogen atoms (F, Cl, Br, or I). Non-limiting examples of lower alkoxy groups include methoxy, ethoxy, n-propoxy, 1-methylethoxy (isopropoxy), n-butoxy, n-pentoxy, t-butoxy, and the like.

The term "cycloalkyl" or "cycloalkyl group" means a stable aliphatic saturated 3- to 15-membered monocyclic or polycyclic monovalent radical consisting solely of carbon and hydrogen atoms which may comprise one or more fused or bridged ring(s), preferably a 3- to 7-membered monocyclic rings. Other exemplary embodiments of cycloalkyl groups include 7- to 10-membered bicyclic rings. Unless otherwise specified, the cycloalkyl ring may be attached at any carbon atom which results in a stable structure and, if substituted, may be substituted at any suitable carbon atom which results in a stable structure. Exemplary cycloalkyl groups include cyclopropyl, cyclobutyl, cyclopentyl, cyclohexyl, cycloheptyl, cyclooctyl, cyclononyl, cyclodecyl, norbornyl, adamantyl, tetrahydronaphthyl (tetralin), 1-decalinyl, bicyclo[2.2.2]octanyl, 1-methylcyclopropyl, 2-methylcyclopentyl, 2-methylcyclooctyl, and the like.

As used herein, the term "aryl" or "aryl group" means an aromatic carbocyclic monovalent or divalent radical. In some embodiments, the aryl group has a number of carbon atoms from 5 to 24 and has a single ring (e.g., phenyl or phenylene), multiple condensed rings (e.g., naphthyl or anthranyl), or multiple bridged rings (e.g., biphenyl). Unless otherwise specified, the aryl ring may be attached at any suitable carbon atom which results in a stable structure and, if substituted, may be substituted at any suitable carbon atom which results in a stable structure. Non-limiting examples of aryl groups include phenyl, naphthyl, anthryl, phenanthryl, indanyl, indenyl, biphenyl, and the like. It may be abbreviated as "Ar".

The term "heteroaryl" or "heteroaryl group" means a stable aromatic monocyclic or polycyclic monovalent or divalent radical, which may comprise one or more fused or bridged ring(s). In some embodiments, the heteroaryl group has 5-24 members, preferably a 5- to 7-membered monocyclic or 7- to 10-membered bicyclic radical. The heteroaryl group can have from one to four heteroatoms in the ring(s) independently selected from nitrogen, oxygen, and sulfur, wherein any sulfur heteroatoms may optionally be oxidized and any nitrogen heteroatom may optionally be oxidized or be quaternized. Unless otherwise specified, the heteroaryl ring may be attached at any suitable heteroatom or carbon atom which results in a stable structure and, if substituted, may be substituted at any suitable heteroatom or carbon atom which results in a stable structure. Non-limiting examples of heteroaryls include furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, triazinyl, indolizinyl, azaindolizinyl, indolyl, azaindolyl, diazaindolyl, dihydroindolyl, dihydroazaindoyl, isoindolyl, azaisoindolyl, benzofuranyl, furanopyridinyl, furanopyrimidinyl, furanopyrazinyl, furanopyridazinyl, dihydrobenzofuranyl, dihydrofuranopyridinyl, dihydrofuranopyrimidinyl, benzothienyl, thienopyridinyl, thienopyrimidinyl, thienopyrazinyl, thienopyridazinyl, dihydrobenzothienyl, dihydrothienopyridinyl, dihydrothienopyrimidinyl, indazolyl, azaindazolyl, diazaindazolyl, benzimidazolyl, imidazopyridinyl, benzthiazolyl, thiazolopyridinyl, thiazolopyrimidinyl, benzoxazolyl, benzoxazinyl, benzoxazinonyl, oxazolopyridinyl, oxazolopyrimidinyl, benzisoxazolyl, purinyl, chromanyl, azachromanyl, quinolizinyl, quinolinyl, dihydroquinolinyl, tetrahydroquinolinyl, isoquinolinyl, dihydroisoquinolinyl, tetrahydroisoquinolinyl, cinnolinyl, azacinnolinyl, phthalazinyl, azaphthalazinyl, quinazolinyl, azaquinazolinyl, quinoxalinyl, azaquinoxalinyl, naphthyridinyl, dihydronaphthyridinyl, tetrahydronaphthyridinyl, pteridinyl, carbazolyl, acridinyl, phenazinyl, phenothiazinyl, and phenoxazinyl, and the like.

In general, the present invention provides a pharmaceutical composition and a method for treating, reducing, or ameliorating an infection of an ear or a portion of an upper respiratory tract.

In one aspect, said infection is selected from the group consisting of otitis, sinusitis, nasopharyngitis, oropharyngitis, and combinations thereof.

In another aspect, a composition of the present invention comprises at least one member of a family of fluoroquinolones that have Formula I or salts thereof, 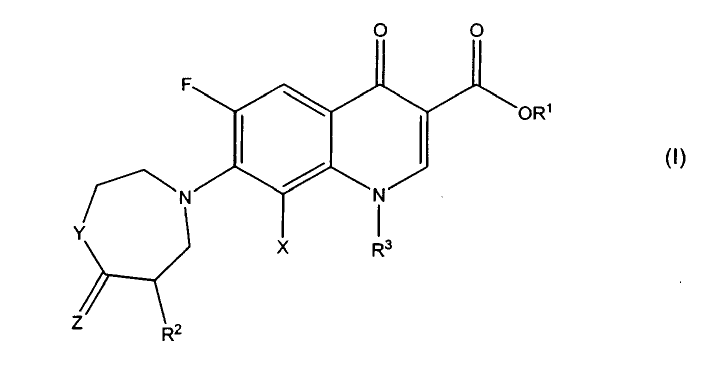 wherein R¹ is selected from the group consisting of hydrogen, unsubstituted lower alkyl groups, substituted lower alkyl groups, cycloalkyl groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, and groups that can be hydrolyzed in living bodies; R² is selected from the group consisting of hydrogen, unsubstituted amino group, and amino groups substituted with one or two lower alkyl groups; R³ is selected from the group consisting of hydrogen, unsubstituted lower alkyl groups, substituted lower alkyl groups, cycloalkyl groups, unsubstituted lower alkoxy groups, substituted lower alkoxy groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, unsubstituted C₅-C₂₄ aryloxy groups, substituted C₅-C₂₄ aryloxy groups, unsubstituted C₅-C₂₄ heteroaryloxy groups, substituted C₅-C₂₄ heteroaryloxy groups, and groups that can be hydrolyzed in living bodies; X is selected from the group consisting of halogen atoms; Y is selected from the group consisting of CH₂, O, S, SO, SO₂, and NR⁴, wherein R⁴ is selected from the group consisting of hydrogen, unsubstituted lower alkyl groups, substituted lower alkyl groups, and cycloalkyl groups; and Z is selected from the group consisting of oxygen and two hydrogen atoms..

In another aspect, a composition of the present invention further comprises a pharmaceutically acceptable carrier.

In one aspect, R¹ is selected from the group consisting of hydrogen, C₁-C₅ (or alternatively, C₁-C₃) substituted and unsubstituted alkyl groups, C₃-C₁₀ (or alternatively, C₃-C₅) cycloalkyl groups, C₅-C₁₄ (or alternatively, C₆-C₁₄, or C₅-C₁₀, or C₆-C₁₀) substituted and unsubstituted aryl groups, C₅-C₁₄ (or alternatively, C₆-C₁₄, or C₅-C₁₀, or C₆-C₁₀) substituted and unsubstituted heteroaryl groups, and groups that can be hydrolyzed in living bodies. In one embodiment, R¹ is selected from the group consisting of C₁-C₅ (or alternatively, C₁-C₃) substituted and unsubstituted alkyl groups.

In another aspect, R² is selected from the group consisting of unsubstituted amino group and amino groups substituted with one or two C₁-C₅ (or alternatively, C₁-C₃) alkyl groups.

In still another aspect, R³ is selected from the group consisting of hydrogen, C₁-C₅ (or alternatively, C₁-C₃) substituted and unsubstituted alkyl groups, C₃-C₁₀ (or alternatively, C₃-C₅) cycloalkyl groups, C₁-C₅ (or alternatively, C₁-C₃) substituted and unsubstituted alkoxy groups, C₅-C₁₄ (or alternatively, C₆-C₁₄, or C₅-C₁₀, or C₆-C₁₀) substituted and unsubstituted aryl groups, C₅-C₁₄ (or alternatively, C₆-C₁₄, or C₅-C₁₀, or C₆-C₁₀) substituted and unsubstituted heteroaryl groups, and C₅-C₁₄ (or alternatively, C₆-C₁₄, or C₅-C₁₀, or C₆-C₁₀) substituted and unsubstituted aryloxy groups. In one embodiment, R³ is selected from the group consisting of C₃-C₁₀ (or alternatively, C₃-C₅) cycloalkyl groups.

In yet another aspect, X is selected from the group consisting of Cl, F, and Br. In one embodiment, X is Cl. In another embodiment, X is F.

In a further aspect, Y is CH₂. In still another aspect, Z comprises two hydrogen atoms.

Some non-limiting members of the family of compounds having Formula I are shown in Table 1. Other compounds of the family not listed in Table 1 are also suitable in selected situations.

**Table 1**

| Some Selected Fluoroquinolones | | | | | | |
|---|---|---|---|---|---|---|
| Compound | R¹ | R² | R³ | X | Y | Z |
| 1 | H | H | CH₃ | Cl | CH₂ | 2 H |
| 2 | H | NH₂ | CH₃ | Cl | CH₂ | 2 H |
| 3 | H | NH₂ | cyclo propyl | Cl | CH₂ | 2 H |
| 4 | H | NH(CH₃) | cyclo propyl | Cl | CH₂ | 2 H |
| 5 | H | N(CH₃)₂ | cyclo propyl | Cl | CH₂ | 2 H |
| 6 | CH₃ | NH₂ | cyclo propyl | Cl | CH₂ | 2 H |
| 7 | C₂H₅ | NH₂ | cyclo propyl | Cl | CH₂ | 2 H |
| 8 | H | NH₂ | cyclo propyl | F | CH₂ | 2 H |
| 9 | H | NH₂ | cyclo propyl | Br | CH₂ | 2 H |
| 10 | H | NH(C₃H₅) | cyclo propyl | Cl | CH₂ | 2 H |
| 11 | H | NH(C₃H₅) | cyclo propyl | F | CH₂ | 2 H |
| 12 | H | NH₂ | cyclo pentyl | Cl | CH₂ | 2 H |
| 13 | H | NH₂ | cyclo propyl | Cl | CH₂ | O |
| 14 | H | NH₂ | cyclo propyl | F | CH₂ | O |
| 15 | H | NH₂ | cyclo propyl | Br | CH₂ | O |
| 16 | H | NH₂ | cyclo propyl | Cl | CH(C₃H₅) | O |
| 17 | CH₃ | NH₂ | cyclo propyl | Cl | CH₂ | O |
| 18 | CH₃ | NH(CH₃) | cyclo propyl | Cl | CH₂ | O |
| 19 | CH₃ | N(CH₃)₂ | cyclo propyl | Cl | CH₂ | O |
| 20 | CH₃ | NH(C₃H₅) | cyclo propyl | Cl | CH₂ | O |

In one embodiment, the fluoroquinolone carboxylic acid has a Formula III.

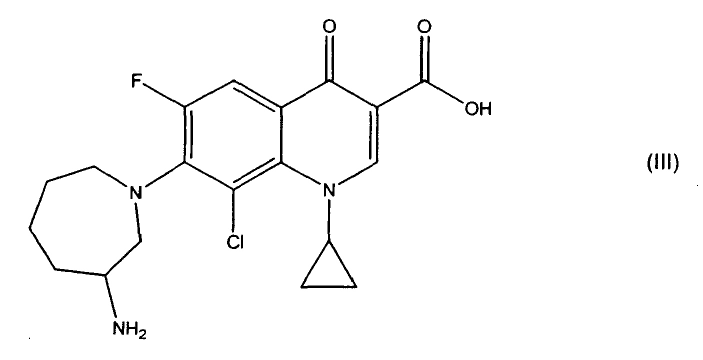

In still another aspect, a composition of the present invention comprises an enantiomer of one of the compounds having Formula I, II, or III.

In still another aspect, a composition of the present invention comprises a mixture of enantiomers of one of the compounds having Formula I, II, or III.

A fluoroquinolone compound disclosed herein can be formulated into a pharmaceutical composition for topical, oral, or systemic administration for the treatment, reduction, amelioration, or prevention of infection of an ear or a portion of the upper respiratory tract. Such a composition comprises a fluoroquinolone compound and a pharmaceutically acceptable carrier for the administration, as can be determined by a person having skill in the art of pharmaceutical formulation for the applications disclosed above. For example, various pharmaceutically acceptable carriers known in the art can be used to formulate a solution, suspension, dispersion, ointment, gel, capsule, or tablet. A fluoroquinolone compound disclosed herein is particularly suitable for a treatment, reduction, amelioration, or prevention of infections of the ear or a portion of the upper respiratory tract caused by bacteria, including, but not being limited to, those bacteria disclosed above. In one embodiment, such a fluroquinolone is formulated into a solution, ointment, suspension, dispersion, or gel.

In one embodiment, a topical composition of the present invention comprises an aqueous solution or suspension. Typically, purified or deionized water is used. The pH of the composition is adjusted by adding any physiologically and otically acceptable pH adjusting acids, bases, or buffers to within the range of about 3 to about 8.5 (or alternatively, or from about 4 to about 7.5, or from about 4 to about 6.5, or from about 5 to about 6.5). Examples of acids include acetic, boric, citric, lactic, phosphoric, hydrochloric, and the like, and examples of bases include sodium hydroxide, sodium phosphate, sodium borate, sodium citrate, sodium acetate, sodium lactate, tromethamine, THAM (trishydroxymethylaminomethane), and the like. Salts and buffers include citrate/dextrose, sodium bicarbonate, ammonium chloride and mixtures of the aforementioned acids and bases. pH buffers are introduced into the composition to maintain a stable pH and to improve product tolerance by the user. In some embodiments, the pH is in the range from about 4 to about 7.5. Biological buffers for various pHs are available, for example, from Sigma-Aldrich. A composition of the present invention can have a viscosity in the range from about 10 to 100,000 centipoise ("cp") or mPa.s. Alternatively, a composition of the present invention can have a viscosity in the range from about 10 to 10,000 cp; or from about 10 to 1,000 cp; or from about 10 to 100 cp; or from about 100 to 1,000 cp; or from about 100 to 10,000 cp; or from about 100 to 50,000 cp; or from about 1,000 to 10,000 cp; or from about 1,000 to 50,000 cp.

In another embodiment, a topical composition of the present invention comprises an ointment, emulsion or cream (such as oil-in-water emulsion), or gel.

Ointments generally are prepared using either (1) an oleaginous base; i.e., one consisting of fixed oils or hydrocarbons, such as white petrolatum or mineral oil, or (2) an absorbent base; i.e., one consisting of an anhydrous substance or substances which can absorb water, for example anhydrous lanolin. Customarily, following formation of the base, whether oleaginous or absorbent, the active ingredient (compound) is added to an amount affording the desired concentration.

Creams are oil/water emulsions. They consist of an oil phase (internal phase), comprising typically fixed oils, hydrocarbons, and the like, such as waxes, petrolatum, mineral oil, and the like, and an aqueous phase (continuous phase), comprising water and any water-soluble substances, such as added salts. The two phases are stabilized by use of an emulsifying agent, for example, a surface active agent, such as sodium lauryl sulfate, hydrophilic colloids, such as acacia colloidal clays, veegum, and the like. Upon formation of the emulsion, the active ingredient (compound) customarily is added in an amount to achieve the desired concentration.

Gels comprise a base selected from an oleaginous base, water, or an emulsion-suspension base. To the base is added a gelling agent which forms a matrix in the base, increasing its viscosity. Examples of gelling agents are hydroxypropyl cellulose, acrylic acid polymers, and the like. Customarily, the active ingredient (compound) is added to the formulation at the desired concentration at a point preceding addition of the gelling agent.

The amount of a fluoroquinolone compound herein disclosed that is incorporated into a formulation of the present invention is not critical; the concentration should be within a range sufficient to permit ready application of the formulation to the affected tissue area in an amount which will deliver the desired amount of compound to the desired treatment site and to provide the desired therapeutic effect. In some embodiments of the present invention, compositions comprise a fluoroquinolone in a concentration in a range from about 0.0001% to 10% by weight (or alternatively, from about 0.001 % to about 5%, or from about 0.01 % to about 5%, or from about 0.01 % to about 2%, or from about 0.01% to about 1%, or from about 0.01% to about 0.7%, by weight).

Moreover, a topical composition of the present invention can contain one or more of the following: surfactants, adjuvants including additional medicaments, antioxidants, tonicity adjusters, preservatives, viscosity modifiers, and the like.

Preservatives may be used to inhibit microbial contamination of the product when it is dispensed in single or multidose containers, and can include, but are not limited to: quaternary ammonium derivatives, (benzalkonium chloride, benzylammonium chloride, cetylmethyl ammonium bromide, cetylpyridinium chloride), benzethonium chloride, organomercury compounds (Thimerosal, phenylmercury acetate, phenylmercury nitrate), methyl and propyl p-hydroxy-benzoates, betaphenylethyl alcohol, benzyl alcohol, phenylethyl alcohol, phenoxyethanol, biguanides (e.g., alexidine, PHMB), and mixtures thereof. These compounds are used at effective concentrations, typically from about 0.005% to about 5% (by weight), depending on the preservative or preservatives selected. The amount of the preservative used should be enough so that the solution is physically stable; i.e., a precipitate is not formed, and antibacterially effective.

The solubility of the components, including a fluoroquinolone having Formula I, II, or III, of the present compositions may be enhanced by a surfactant or other appropriate co-solvent in the composition or complexing agents such as cyclodextrins (hydroxypropyl, hydroxyethyl, glucosyl, maltosyl and maltotriosyl derivatives of α-, β-, and γ-cyclodextrin), EDTA, citrate buffer, and succinate buffer. In one embodiment, the composition comprises 0.1% to 20% hydroxypropyl- β-cyclodextrin; alternatively, 1% to 15% (or 2% to 10%) hydroxypropyl- β-cyclodextrin. Co-solvents include polysorbates (for example, polysorbate 20, 60, and 80), polyoxyethylene/polyoxypropylene surfactants (e.g., Pluronic® F68, F84, F127, and P103), cyclodextrin, fatty-acid triglycerides, glycerol, polyethylene glycol, other solubility agents such as Octoxynol 40 and Tyloxapol, or other agents known to those skilled in the art and mixtures thereof. The amount of solubility enhancer used will depend on the amount of fluoroquinolone in the composition, with more solubility enhancer used for greater amounts of fluoroquinlones. Typically, solubility enhancers are employed at a level of from 0.0 1 % to 20% (alternatively, 0.1% to 5%, or 0.1% to 2%) by weight depending on the ingredient.

The use of viscosity enhancing agents to provide the compositions of the invention with viscosities greater than the viscosity of simple aqueous solutions may be desirable to increase absorption of the active compounds by the target tissues or to increase the retention time in the ear. Such viscosity enhancing agents include, for example, polyvinyl alcohol, polyvinyl pyrrolidone, methyl cellulose, hydroxypropylmethyl cellulose, hydroxyethyl cellulose, carboxymethyl cellulose, hydroxypropyl cellulose or other agents know to those skilled in the art. Such agents are typically employed at a level of from 0.01% to 10% (alternatively, 0.1% to 5%, or 0.1% to 2%) by weight.

Suitable surfactants include polyvinyl pyrolidone, polyvinyl alcholol, polyethylene glycol, ethylene glycol, and propylene glycol. Other surfactants are polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween® 80, Tween® 60, Tween® 20), poloxamers (synthetic block polymers of ethylene oxide and propylene oxide, such as those commonly known by their trade names of Pluronic®; e.g., Pluronic® F127 or Pluronic® F108)), or poloxamines (synthetic block polymers of ethylene oxide and propylene oxide attached to ethylene diamine, such as those commonly known by their trade names of Tetronic®; e.g., Tetronic® 1508 or Tetronic® 908, etc., other nonionic surfactants such as Brij®, Myrj®, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). The surfactant helps a topical formulation to spread on the surface of narrow passages.

It is often that an infection is followed by inflammation. Therefore, in another aspect, a composition of the present invention further comprises an anti-inflammatory agent. Anti-inflammatory agents include the well-known glucocorticosteroids and the non-steroidal anti-inflammatory drugs ("NSAIDs").

Non-limiting examples of the glucocorticosteroids are: 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortarnate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, their physiologically acceptable salts, combinations thereof, and mixtures thereof.

The preferred glucocorticoids for otic use include dexamethasone, loteprednol, rimexolone, prednisolone, fluorometholone, hydrocortisone, and their derivatives. The preferred glucocorticoids for nasal use include mometasone, fluticasone, beclomethasone, flunisolide, triamcinolone, budesonide, and their derivatives.

Non-limiting examples of the NSAIDs are: aminoarylcarboxylic acid derivatives (e.g., enfenamic acid, etofenamate, flufenamic acid, isonixin, meclofenamic acid, mefenamic acid, niflumic acid, talniflumate, terofenamate, tolfenamic acid), arylacetic acid derivatives (e.g., aceclofenac, acemetacin, alclofenac, amfenac, amtolmetin guacil, bromfenac, bufexamac, cinmetacin, clopirac, diclofenac sodium, etodolac, felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, zomepirac), arylbutyric acid derivatives (e.g., bumadizon, butibufen, fenbufen, xenbucin), arylcarboxylic acids (e.g., clidanac, ketorolac, tinoridine), arylpropionic acid derivatives (e.g., alminoprofen, benoxaprofen, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprolen, pirprofen, pranoprofen, protizinic acid, suprofen, tiaprofenic acid, ximoprofen, zaltoprofen), pyrazoles (e.g., difenamizole, epirizole), pyrazolones (e.g., apazone, benzpiperylon, feprazone, mofebutazone, morazone, oxyphenbutazone, phenylbutazone, pipebuzone, propyphenazone, ramifenazone, suxibuzone, thiazolinobutazone), salicylic acid derivatives (e.g., acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide o-acetic acid, salicylsulfuric acid, salsalate, sulfasalazine), thiazinecarboxamides (e.g., ampiroxicam, droxicam, isoxicam, lornoxicam, piroxicam, tenoxicam), ε-acetamidocaproic acid, S-(5'-adenosyl)-L-methionine, 3-amino-4-hydroxybutyric acid, amixetrine, bendazac, benzydamine, α-bisabolol, bucolome, difenpiramide, ditazol, emorfazone, fepradinol, guaiazulene, nabumetone, nimesulide, oxaceprol, paranyline, perisoxal, proquazone, superoxide dismutase, tenidap, zileuton, their physiologically acceptable salts, combinations thereof, and mixtures thereof.

Other non-steroidal anti-inflammatory agents include the cyclooxygenase type II selective inhibitors, such as Celecoxib, and etodolac; PAF antagonists, such as apafant, bepafant, minopafant, nupafant, and modipafant; PDE IV inhibitors, such as ariflo, torbafylline, rolipram, filaminast, piclamilast, cipamfylline, and roflumilast; inhibitors of cytokine production, such as inhibitors of the NF-κB transcription factor; or other anti-inflammatory agents known to those skilled in the art.

The concentrations of the anti-inflammatory agents contained in the compositions of the present invention will vary based on the agent or agents selected and the type of inflammation being treated. The concentrations will be sufficient to reduce inflammation in the targeted otic or upper respiratory tract tissues following topical application of the compositions to those tissues. Such concentrations are typically in the range from about 0.0001 to about 3% by weight (or alternatively, from about 0.01 to about 2%, or from about 0.05% to about 1%, by weight).

Bacterial pathogens that have been isolated from cases of ear infection include *Pseudomonas aeruginosa, Staphylococcus aureus, Streptococcus pneumoniae, Streptococcus pyrogenes, Streptococcus faecalis, Haemophilus influenzae, Moraxella catarahalis, Escherichia coli, Proteus* species, *Klebsiella* species, and *Enterococcus* species. Several of these species from the isolates have been found to be resistant to a number of antimicrobial drugs. For example, a published study of antimicrobial-resistant pathogens in middle-ear fluid of children with acute otitis media shows that thirty percent of the *S. pneumoniae* isolates were intermediately or fully resistant, and eight percent fully resistant, to penicillin; ten percent of the isolates were resistant to amoxicillin or amoxicillin-clavulanate. M.R. Jacobs et al., Antimicrobial Agents and Chemotherapy, Vol. 42, No. 3, 589 (1998). The same study shows that thirty percent of *H. influenzae* isolates produced β-lactamase, and thus, were expected to be resistant to penicillin.

Bacterial pathogens that have been isolated from cases of upper respiratory infection include *Staphylococcus aureus, Staphylococcus epidermidis, Streptococcus pneumoniae, Streptococcus pyrogenes, Haemophilus influenzae, Peptostreptococcus* species, and *Bacteroides* species.

Anti-bacterial activity of the compound having Formula III was tested against several Gram-negative reference bacteria strains and compared to the anti-bacterial activity of three commercially available antibiotics (nadifloxacin, ofloxacin, and sparfloxacin). The results are shown in Table 2 as MIC₉₀ values (minimum concentration of the active compound required to inhibit ninety percent of the growth of a specified pathogen, in µg/ml).

**Table 2**

| Comparison of *In vitro* Anti-bacterial Activity of Compound Having Formula III, Nadifloxacin, Ofloxacin, and Sparfloxacin Against Gram-negative Bacteria | | | | |
|---|---|---|---|---|
| Strain | MIC₉₀ (µg/mL) | | | |
| | Compound Having Formula III | Nadifloxacin | Ofloxacin | Sparfloxacin |
| *Escherichia coli* (O-1) | 0.1 | 0.2 | 0.05 | 0.012 |
| *Klebsiella pneumoniae* (IFO 13541) | 0.024 | 0.1 | 0.024 | 0.012 |
| *Salmonella typhimurium* (TD) | 0.05 | 0.2 | 0.05 | 0.012 |
| *Shigella flexneri* (2b) | 0.006 | 0.006 | 0.006 | 0.006 |
| *Enterobacter aerogenes* (IFO 13534) | 0.39 | 1.56 | 0.2 | 0.1 |
| *Serratia marcenscens* (NHL) | 0.2 | 1.56 | 0.1 | 0.2 |
| *Proteus mirabilis* (IFO 13300) | 0.1 | 0.1 | 0.05 | 0.1 |
| *Proteus rettgeri* | 0.39 | 0.39 | 0.2 | 0.2 |
| *Acinetobacter calcoaceticus* (IFO 12552) | 0.39 | 0.78 | 0.39 | 0.024 |
| *Pseudomonas aeruginosa* (IFO 13736) | 1.56 | 3.13 | 1.56 | 0.78 |
| *Pseudomonas aeruginosa* (ATCC 27853) | 1.56 | 6.25 | 3.13 | 0.78 |

Anti-bacterial activity of the compound having Formula III was tested against several Gram-positive reference bacteria strains and compared to the anti-bacterial activity of three commercially available antibiotics (nadifloxacin, ofloxacin, and sparfloxacin). The results are shown in Table 3 as MIC₉₀ values.

**Table 3**

| Comparison of *In vitro* Anti-bacterial Activity of Compound Having Formula III, Nadifloxacin, Ofloxacin, and Sparfloxacin Against Gram-positive Bacteria | | | | |
|---|---|---|---|---|
| Strain | MIC₉₀ (µg/mL) | | | |
| | Compound Having Formula III | Nadifloxacin | Ofloxacin | Sparfloxacin |
| *Bacillis subtilis* (ATCC 6633) | 0.012 | 0.006 | 0.05 | 0.024 |
| *Staphylococcus aureus* (ATCC 25933) | 0.05 | 0.05 | 0.39 | 0.1 |
| *Staphylococcus aureus* (FDA 209P) | 0.012 | 0.024 | 0.2 | 0.024 |
| *Staphylococcus aureus* (Smith) | 0.006 | 0.024 | 0.2 | 0.05 |
| *Staphylococcus epidermidis* (ATCC 12228) | 0.05 | 0.1 | 0.78 | 0.2 |
| *Sarcina lutea* (ATCC 9341) | 0.1 | 0.39 | 3.13 | 1.56 |
| *Streptococcus faecalis* (IFO 12964) | 0.1 | 0.78 | 1.56 | 0.78 |
| *Streptococcus faecalis* (ATCC 29212) | 0.2 | 0.78 | 1.56 | 0.39 |
| *Streptococcus pyogenes* (Cook) | 0.1 | 0.78 | 0.78 | 0.39 |
| *Streptococcus pyogenes* (IID 698) | 0.1 | 0.1 | 0.39 | 0.2 |
| *Streptococcus pneumoniae* (IID 553) | 0.1 | 0.78 | 1.56 | 0.39 |
| *Streptococcus pneniae* (IID 554) | 0.2 | 0.78 | 1.56 | 0.39 |

Anti-bacterial activity of the compound having Formula III was tested against some methicillin-resistant *Staphylococcus aureus* bacteria strains and compared to the anti-bacterial activity of three commercially available antibiotics (nadifloxacin, ofloxacin, and sparfloxacin). The results are shown in Table 4 as MIC₉₀ values.

**Table 4**

| Comparison of *In vitro* Anti-bacterial Activity of Compound Having Formula III, Nadifloxacin, Ofloxacin, and Sparfloxacin Against Methicillin-resistant *Staphylococcus aureus* Isolates | | | | |
|---|---|---|---|---|
| Strain | MIC₉₀ (µg/mL) | | | |
| | Compound Having Formula III | Nadifloxacin | Ofloxacin | Sparfloxacin |
| *Staphylococcus atreus* (ATCC 33591) | 0.012 | 0.006 | 0.05 | 0.024 |
| *Staphylococcus atreus* (ATCC 33592) | 0.05 | 0.05 | 0.39 | 0.1 |
| *Staphylococcus atreus* (ATCC 33593) | 0.012 | 0.024 | 0.2 | 0.024 |
| *Staphylococcus aureus* (No. 395) | 0.006 | 0.024 | 0.2 | 0.05 |
| *Staphylococcus aureus* (No. 415) | 0.05 | 0.1 | 0.78 | 0.2 |
| *Staphylococcus aureus* (No. 419) | 0.1 | 0.39 | 3.13 | 1.56 |
| *Staphylococcus atreus* (No. 420) | 0.1 | 0.78 | 1.56 | 0.78 |
| *Staphylococcus atreus* (No. 421) | 0.2 | 0.78 | 1.56 | 0.39 |

Anti-bacterial activity of the compound having Formula III was tested against selected anaerobic reference bacteria and compared to the anti-bacterial activity of three commercially available antibiotics (nadifloxacin, ofloxacin, and sparfloxacin). The results are shown in Table 5 as MIC₉₀ values.

**Table 5**

| Comparison of *In* vitro Anti-bacterial Activity of Compound Having Formula III, Nadifloxacin, Ofloxacin, and Sparfloxacin Against Anaerobic Bacteria | | | | |
|---|---|---|---|---|
| Strain | MIC₉₀ (µg/mL) | | | |
| | Compound Having Formula III | Nadifloxacin | Ofloxacin | Sparfloxacin |
| *Clostridium perfringens* (KZ 210) | 0.2 | 0.2 | 0.78 | 0.39 |
| *Peptostreptococcus micros* (GIFU 7824) | 0.2 | 0.39 | 0.78 | 0.78 |
| *Peptostreptococcus magnum* (GAI 0664) | 0.05 | 0.2 | 0.39 | 0.1 |
| *Propionibacterium acnes* (GAI 5419) | 0.2 | 0.2 | 0.78 | 0.39 |
| *Propionibacterium acnes* (ATCC 6919) | 0.2 | 0.2 | 0.78 | 0.39 |
| *Propionibacterium acnes* (ATCC 11828) | 0.39 | 0.78 | 1.56 | 0.78 |
| *Bacteroides fragillis* (GAI 0675) | 0.1 | 1.56 | 1.56 | 0.78 |
| *Bacteroides thetaiotaomicron* (GAI 0659) | 0.2 | 1.56 | 3.13 | 0.78 |
| *Bacteroides vulgatus* (GAI 0673) | 0.1 | 0.78 | 3.13 | 0.78 |
| *Veillonella parvula* (GAI 5602) | 0.1 | 0.78 | 0.39 | 0.2 |

Anti-bacterial activity of the compound having Formula III was tested against some ophthalmologic clinical bacteria isolates and compared to the anti-bacterial activity of three commercially available antibiotics (nadifloxacin, ofloxacin, and ciprofloxacin). As disclosed above, most of these bacteria strains are also relevant to infections of the ear and upper respiratory tract. The results are shown in Table 6 as MIC₉₀ values.

**Table 6**

| Comparison of *In vitro* Anti-bacterial Activity of Compound Having Formula III, Nadifloxacin, Ofloxacin, and Ciprofloxacin Against Some Clinical Bacteria Isolates | | | | |
|---|---|---|---|---|
| Strain | MIC₉₀ (µg/mL) | | | |
| | Compound Having Formula III | Norfloxacin | Ofloxacin | Ciprofloxacin |
| Gram-negative | | | | |
| *Moraxella* species | 0.13 | 0.25 | 0.13 | ≤ 0.06 |
| *Pseudomonas aeruginosa* | 4 | 4 | 4 | 0.5 |
| Haemophilus influenzae | ≤ 0.06 | ≤ 0.06 | ≤ 0.06 | ≤ 0.06 |
| Gram-positive | | | | |
| *Staphylococcus aureus* | I | > 128 | 32 | 64 |
| Coagulase-negative *Staphylococcus* | 0.5 | 128 | 8 | 16 |
| *Streptococcus pneumoniae* | 0.13 | 8 | 2 | 1 |
| *Corynebacterium* species | 2 | 16 | 32 | 8 |
| *Propionibacterium acnes* | 0.25 | 4 | 1 | 0.5 |
| Antibiotic-resistant Organisms | | | | |
| Ofloxacin-resistant *Enterobacteriaceae* | 16 | 128 | 64 | 32 |
| Ofloxacin -resistant *Staphylococcus aureus* | 8 | > 128 | > 128 | > 128 |
| Gentamycin-resistant *Staphylococcus aureus* | 4 | 128 | 128 | 128 |
| Gentamycin-resistant *Pseudomonas aeroginosa* | 32 | 128 | 64 | 32 |
| Penicillin-resistant Streptococcus pneumoniae | 0.13 | 8 | 2 | 1 |

The results show that the compound having Formula III is effective against bacteria, including some antibiotic-resistant strains, which have been found in cases of infection of the ear and upper respiratory tract. Although the applicants do not wish to be bound by any particular theory, they believe that the unique moieties on the fluoroquinolones disclosed herein provide their advantageous antibacterial property and are effective for the treatment, reduction, amelioration, or prevention of infection of the ear, including otitis externa and otitis media, and infection of the upper respiratory tract, including sinusitis, nasopharyngitis, and oropharyngitis.

In another aspect, a composition of the present invention (especially one having low viscosity) is useful to treat, reduce, ameliorate, or prevent otitis media in patients with tympanic tubes.

The following examples are provided to further illustrate non-limiting compositions of the present invention, and methods of preparing such composition, for the treatment, reduction, amelioration, or prevention of infection of the ear and/or upper respiratory tract.

### EXAMPLE 1: Otic or Nasal Solution

| Ingredient | Amount (% by weight) |
|---|---|
| Compound having Formula III | 0.2 |
| Hydroxypropylmethyl cellulose ("HPMC") | 0.5 |
| Benzakonium chloride ("BAK") | 0.01 |
| Pluroruc® F127 | 0.1 |
| Polysorbate 60 | 0.1 |
| Cetyl stearyl alcohol | 0.01 |
| EDTA | 0.1 |
| NaCl | 0.25 |
| Phosphate buffer (0.05M, pH = 5.0) | q.s. to 100 |

An appropriate proportion (shown in the above table) of Pluronic® F127 is added to phosphate buffer in a stainless steel jacketed vessel equipped with a stirring mechanism. An appropriate amount of BAK is added to the buffer solution while mixing three to ten minutes. The mixture may be heated up to 75 °C. Then, an appropriate amount of the compound having Formula III is added to the contents of the vessel over a period of three to five minutes while mixing continues until the compound is completely dissolved. EDTA and NaCl are then added to the mixture while mixing continues for five more minutes. To a second vessel containing an appropriate amount of distilled water, also equipped with a stirring mechanism, heated to about 75° C, an appropriate amount of HPMC is added, over a period of three to five minutes to form a uniform dispersion. Polysorbate 60 and cetyl stearyl alcohol are added to the mixture in the second vessel. The contents of the second vessel are cooled to about room temperature and then added to the mixture in the first vessel. The resulting mixture is cooled to 25 to 30° C, if it is not already in this temperature range. The final composition is packaged in appropriate containers.

### EXAMPLE 2: Otic or Nasal Solution

A procedure similar to that of Example 1 is used to produce this solution.

| Ingredient | Amount (% by weight) |
|---|---|
| Compound having Formula III | 0.35 |
| Mannitol | 4.5 |
| Benzakonium chloride ("BAK") | 0.005 |
| Polysorbate 80 | 0.1 |
| EDTA | 0.05 |
| Sodium acetate | 0.03 |
| Acetic acid | 0.04 |
| Purified water | q.s. to 100 |

### EXAMPLE 3: Otic or Nasal Solution

A procedure similar to that of Example I is used to produce this solution having the following composition.

| Ingredient | Amount (% by weight) |
|---|---|
| Compound having Formula III | 0.2 |
| Dexamethasone | 0.1 |
| Hydroxypropylmethyl cellulose ("HPMC") | 0.5 |
| Alexidine | 0.01 |
| Brij® surfactant | 0.1 |
| EDTA | 0.1 |
| Citrate buffer (0.02M sodium citrate, pH = 5.0) | q.s. to 100 |

### EXAMPLE 4: Otic or Nasal Solution

A procedure similar to that of Example 1 is used to produce this solution having the following composition.

| Ingredient | Amount (% by weight) |
|---|---|
| Compound 8 of Table 1 | 0.3 |
| Colecoxib | 0.15 |
| Propylene glycol | 0.5 |
| Alexidine | 0.01 |
| Tyloxapol | 0.1 |
| EDTA | 0.1 |
| Citrate buffer (0.02M sodium citrate, pH = 5.0) | q.s. to 100 |

### EXAMPLE 5: Otic or Nasal Suspension

A procedure similar to that of Example 1 is used to produce this suspension having the following composition.

| Ingredient | Amount (% by weight) |
|---|---|
| Compound having Formula III | 0.3 |
| Triamcinolone, micronized USP | 0.2 |
| Hydroxyethyl cellulose | 0.25 |
| BAK | 0.01 |
| Tyloxapol | 0.05 |
| EDTA | 0.01 |
| NaCl | 0.3 |
| Na₂SO₄ | 1.2 |
| Sulfuric acid and/or NaOH | q.s. for pH adjustment to 5.5 |
| Citrate buffer (0.02M sodium citrate, pH = 5.0) | q.s. to 100 |

### EXAMPLE 6: Otic or Nasal Emulsion

A modification of the procedure of Example 1 is used to produce this emulsion having the composition shown in the table immediately below.

Polysorbate 60 (Tween 60) is added to water in a first sterilized stainless steel jacketed vessel, equipped with a stirring mechanism, at a temperature of 50° C to 60° C in amounts corresponding the proportions shown in the table below. The resulting aqueous solution is heated to 61° C to 75° C. At a temperature of 66° C, benzyl alcohol (a preservative) is added to the aqueous solution while mixing three to ten minutes. At a temperature of 75° C, appropriate amounts of the compound having Formula III and loteprednole etabonate are added to Mygliol oil in a second sterilized vessel, also equipped with a stirring mechanism, over a period of three to five minutes while stirring continues. Sorbitan monostearate and cetyl stearyl alcohol are added to the oil mixture. The resulting oil mixture is heated to a temperature in the range from 62° C to 75° C. The oil mixture is then added with vigorous mixing to the aqueous solution in the first vessel at a temperature of 66° C over a period of three to five minutes. Sodium sulfate and sulfuric acid and/or sodium hydroxide are added to the mixture to adjust pH to 5.5. The resulting composition is cooled to 35° C to 45° C and homogenized by mixing with a high shear emulsifier or running through a homogenizer. The composition is further cooled to 25° C to 30° C. The final composition is packaged in appropriate containers.

| Ingredient | Amount (% by weight) |
|---|---|
| Compound having Formula III | 0.5 |
| Loteprednol etabonate | 0.2 |
| Polysorbate 60 | 1 |
| Sorbitan monostearate (an emulsifier) | 1.5 |
| Cetyl stearyl alcohol (an emulsion stabilizer) | 1.5 |
| Benzyl alcohol | 0.5 |
| Miglyol oil | 14.5 |
| Na₂SO₄ | 1.2 |
| Sulfuric acid and/or NaOH | q.s. for pH adjustment to 5.5 |
| Purified water | q.s. to 100 |

Typically, the oil used in an emulsion is a non-irritating emollient oil. Illustrative but non-limiting examples thereof include a mineral oil, vegetable oil, and a reformed vegetable oil of known composition. More specific but non-limiting examples of the oil can be selected from the group consisting of peanut oil, sesame seed oil, cottonseed oil, and a medium chain (C₆ to C₁₂) triglycerides (e.g., Miglyol Neutral Oils 810, 812, 818, 829, 840, etc., available from Huls America Inc.). Typical emulsifiers employed can be selected from the group consisting of sorbitan monostearate and Tween 60 (also known as Polysorbate 60). Preferably, the emulsifiers are nonionic. The emulsifiers can be employed in an amount of 1.5 to 6.5% by weight of the composition, and preferably, 3 to 5% by weight of the composition. The hydrophobic phase of the emulsion can be in an amount of 15 to 25% by weight of the composition, and preferably, 18 to 22% by weight of the composition.

### EXAMPLE 7: Otic or Nasal Emulsion

A procedure similar to that of Example 6 is used to produce this emulsion having the following composition.

| Ingredient | Amount (% by weight) |
|---|---|
| Compound 13 of Table 1 | 0.5 |
| Triamcinolone, micronized USP | 0.2 |
| Polysorbate 60 | 1 |
| Sorbitan monostearate | 1.5 |
| Cetyl stearyl alcohol | 1.5 |
| Benzyl alcohol | 0.5 |
| Miglyol oil | 14.5 |
| Na₂SO₄ | 1.2 |
| Sulfuric acid and/or NaOH | q.s. for pH adjustment to 5.5 |
| Purified water | q.s. to 100 |

### EXAMPLE 8: Otic or Nasal Ointment

A procedure similar to that of Example 1 is used to produce this ointment having the following composition.

| Ingredient | Amount (% by weight) |
|---|---|
| Compound having Formula III | 0.3 |
| White petrolatum USP | 50 |
| Propylene glycol | 5 |
| Glycerin | 5 |
| Tween 20 | 2 |
| Vitamin E | 1 |
| BAK | 0.1 |
| Mineral oil | q.s. to 100 |

### EXAMPLE 9: Otic or Nasal Ointment

A procedure similar to that of Example 1 is used to produce this ointment having the following composition.

| Ingredient | Amount (% by weight) |
|---|---|
| Compound having Formula III | 0.3 |
| Dexamethasone | 0.15 |
| White petrolatum USP | 50 |
| Propylene glycol | 5 |
| Glycerin | 5 |
| Tween 20 | 2 |
| Vitamin E | I |
| Vitamin D | 0.5 |
| BAK | 0.1 |
| Mineral oil | q.s. to 100 |

The present invention also provides a means for treating, reducing, ameliorating, or preventing infection of the ear or upper respiratory tract. In one aspect, it is described administering one or more drops of a composition of the present invention to the ear canal, nasal cavity, or back of the throat of a subject who has indication of infection or whose risk of infection is indicated. A composition of the present invention can also be formulated into a spray, which can be administered into the otic or nasal cavity of such a subject.

## Claims

1. A composition for use in treating, reducing, ameliorating, or preventing infection of an ear or a portion of an upper respiratory tract of a subject, the composition comprising a fluoroquinolone having Formula I or salts thereof, wherein the fluoroquinolone is present in an amount effective to treat, reduce, ameliorate, or prevent said infection, 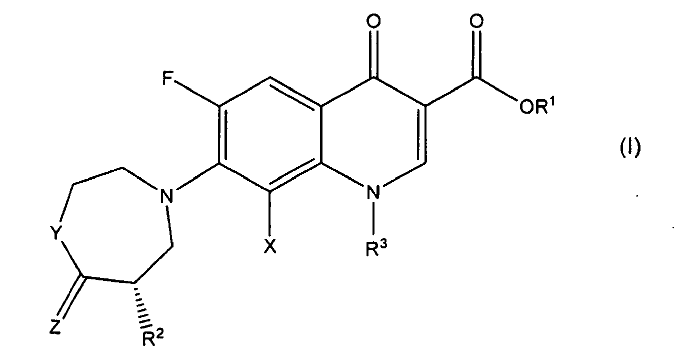 wherein R¹ is selected from the group consisting of hydrogen, unsubstituted C₁-C₁₅ linear- or branched-chain saturated aliphatic hydrocarbon monovalent groups, substituted C₁-C₁₅ linear- or branched-chain saturated aliphatic hydrocarbon monovalent groups, cycloalkyl groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, and groups that can be hydrolyzed in living bodies; R² is selected from the group consisting of hydrogen, unsubstituted amino group, and amino groups substituted with one or two substituted or unsubstituted C₁-C₁₅ linear- or branched-chain saturated aliphatic hydrocarbon monovalent groups; R³ is selected from the group consisting of hydrogen, unsubstituted C₁-C₁₅ linear- or branched-chain saturated aliphatic hydrocarbon monovalent groups, substitutedC₁-C₁₅ linear- or branched-chain saturated aliphatic hydrocarbon monovalent groups, cycloalkyl groups, unsubstituted C₁-C₁₅ linear- or branched-chain saturated aliphatic alkoxy monovalent groups, substituted C₁-C₁₅ linear- or branched-chain saturated aliphatic alkoxy monovalent groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, unsubstituted C₅-C₂₄ aryloxy groups, substituted C₅-C₂₄ aryloxy groups, unsubstituted C₅-C₂₄ heteroaryloxy groups, and substituted C₅-C₂₄ heteroaryloxy groups; X is selected from the group consisting of halogen atoms; Y is selected from the group consisting of CH₂, O, S, SO, SO₂ and NR⁴, wherein R⁴ is selected from the group consisting of hydrogen, unsubstituted C₁-C₁₅ linear- or branched-chain saturated aliphatic hydrocarbon monovalent groups, substituted C₁-C₁₅ linear- or branched-chain saturated aliphatic hydrocarbon monovalent groups, and cycloalkyl groups; and Z is selected from the group consisting of oxygen and two hydrogen atoms; and wherein the composition is capable of inhibiting a growth or survival of a microorganism causing said infection.

2. The composition for use of claim 1, wherein R¹ is selected from the group consisting of hydrogen, C₁-C₅ substituted and unsubstituted alkyl groups, C₃-C₁₀ cycloalkyl groups, C₆-C₁₄ substituted and unsubstituted aryl groups, C₅-C₁₄ substituted and unsubstituted heteroaryl groups, and groups that can be hydrolyzed in living bodies; R² is selected from the group consisting of unsubstituted amino group and amino groups substituted with one or two C₁-C₅ alkyl groups; R³ is selected from the group consisting of hydrogen, C₁-C₅ substituted and unsubstituted alkyl groups, C₃-C₁₀ cycloalkyl groups, C₁-C₅ substituted and unsubstituted alkoxy groups, C₅-C₁₄ substituted and unsubstituted aryl groups, C₅-C₁₄ substituted and unsubstituted heteroaryl groups, and C₅-C₁₄ substituted and unsubstituted aryloxy groups; and X is selected from the group consisting of Cl, F, and Br.

3. The composition for use of claim 1, wherein R¹ is selected from the group consisting of hydrogen, C₁-C₅ substituted and unsubstituted alkyl groups and groups that can be hydrolyzed in living bodies; R² is selected from the group consisting of unsubstituted amino group and amino groups substituted with one or two C₁-C₅ alkyl groups; R³ is selected from the group consisting of C₃-C₁₀ cycloalkyl groups; X is selected from the group consisting of Cl and F; Y comprises CH₂; and Z comprises two hydrogen atoms.

4. The composition for use of claim 1, wherein the fluoroquinolone is present in an amount from 0.0001 to 10% by weight.

5. The composition for use of claim 4, wherein the fluoroquinolone is present in an amount from 0.01 to 5% by weight.

6. The composition for use of claim 5, wherein the fluoroquinolone is present in an amount from 0.01 to 1% by weight.

7. The composition for use of claim 5, further comprising an anti-inflammatory agent.

8. The composition for use of claim 7, wherein said anti-inflammatory agent is selected from the group consisting of glucocorticosteroids, non-steroidal anti-inflammatory drugs, inhibitors of cytokine production, and mixtures thereof.

9. The composition for use of claim 4, further comprising a material selected from the group consisting of buffers, surfactants, viscosity modifiers, tonicity adjusting agents, preservatives, antioxidants, additional medicaments, and combinations thereof.

10. The composition for use of claim 8, further comprising a material selected from the group consisting of buffers, surfactants, viscosity modifiers, tonicity adjusting agents, preservatives, antioxidants, additional medicaments, and combinations thereof.

11. The composition for use of claim 4, wherein the fluoroquinolone has Formula III

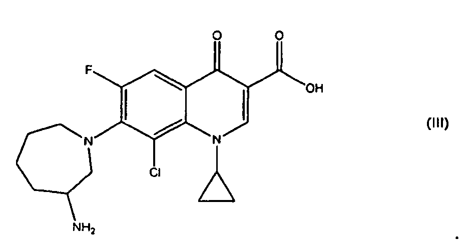

12. The composition for use of claim 7, wherein the fluoroquinolone has Formula III

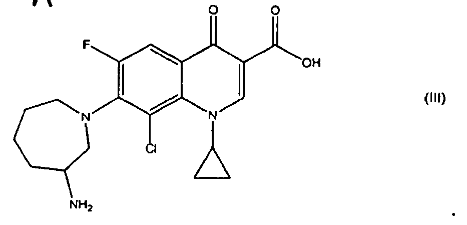

13. The composition for use of claim 1, comprising a single enantiomer of the fluoroquinolone having Formula I.

14. The composition for use of claim 11, comprising a single enantiomer of the fluoroquinolone having Formula III.

15. The composition for use of claim 12, comprising a single enantiomer of the fluoroquinolone having Formula III.

16. The composition for use of claim 4, wherein the composition is in a form of a solution, cream, emulsion, suspension, ointment, or gel.

17. The composition for use of claim 1, wherein the composition is for topical, oral, or systemical administration.

18. The composition for use of claim 17, wherein the composition is for topical administration.

19. The composition for use of claim 18, wherein the infection is otitis externa, otitis media, or a combination thereof.

20. The composition for use of claim 18, wherein the infection is selected from the group consisting of sinusitis, nasopharyngitis, oropharyngitis, and combinations thereof.

21. The composition for use of claim 1, wherein the fluoroquinolone has Formula III .

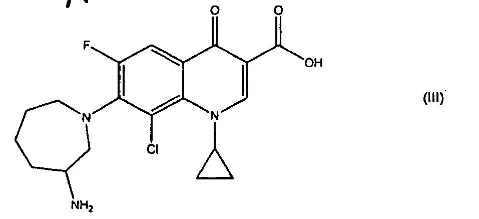

22. The composition for use of claim 21, wherein the composition is for topical, oral, or systemical administration.

23. The composition for use of claim 22, wherein the composition for topical administration.

24. The composition for use of claim 23, wherein the infection is otitis externa, otitis media, or a combination thereof.

25. The composition for use of claim 23, wherein the infection is selected from the group consisting of sinusitis, nasopharyngitis, oropharyngitis, and combinations thereof.

26. The composition for use of claim 1, wherein said microorganism is bacteria that is resistant to an antibiotic.

27. The composition for use of claim 21, wherein the composition is capable of inhibiting a growth or survival of a microorganism that is resistant to an antibiotic.

## Patentansprüche

1. Eine Zusammensetzung zur Verwendung bei der Behandlung, Reduzierung, Verbesserung oder Vorbeugung einer Infektion eines Ohres oder eines Bereichs der oberen Atemwege eines Subjektes, wobei die Zusammensetzung ein Fluoroquinolon der Formel I oder Salze davon umfasst, wobei das Fluoroquinolon in Mengen, die für die Behandlung, Reduzierung, Verbesserung oder Vorbeugung besagter Infektion wirksam sind, vorhanden ist, 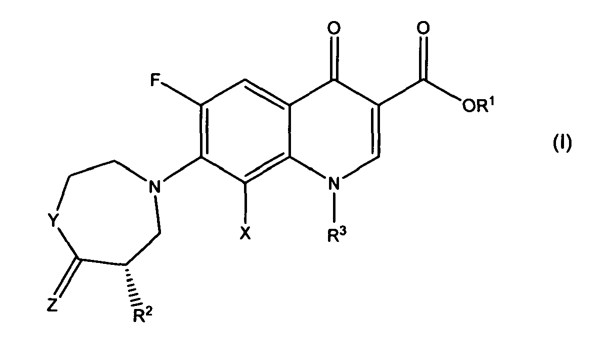 wobei R¹ ausgewählt ist aus der Gruppe bestehend aus Wasserstoff, unsubstituierten linearen oder verzweigten monovalenten gesättigten aliphatischen C₁-C₁₅ Kohlenwasserstoff-Gruppen, substituierten linearen oder verzweigten monovalenten gesättigten aliphatischen C₁-C₁₅ Kohlenwasserstoff-Gruppen, Cycloalkyl-Gruppen, unsubstituierten C₅-C₂₄ Aryl-Gruppen, substituierten C₅-C₂₄ Aryl-Gruppen, unsubstituierten C₅-C₂₄ Heteroaryl-Gruppen, substituierten C₅-C₂₄ Heteroaryl-Gruppen und Gruppen welche in lebenden Körpern hydrolisiert werden können; R² ausgewählt ist aus der Gruppe bestehend aus Wasserstoff, unsubstituierten Amino-Gruppen und Amino-Gruppen, die mit einer oder zwei substituierten oder unsubstituierten linearen oder verzweigten monovalenten gesättigten aliphatischen C₁-C₁₅ Kohlenwasserstoff-Gruppen substituiert sind; R³ ausgewählt ist aus der Gruppe bestehend aus Wasserstoff, unsubstituierten linearen oder verzweigten monovalenten gesättigten aliphatischen C₁-C₁₅ Kohlenwasserstoff Gruppen, substituierten linearen oder verzweigten monovalenten gesättigten aliphatischen C₁-C₁₅ Kohlenwasserstoff-Gruppen, Cycloalkyl-Gruppen, unsubstituierten linearen oder verzweigten monovalenten gesättigten aliphatischen C₁-C₁₅ Alkoxy-Gruppen, substituierten linearen oder verzweigten monovalenten gesättigten aliphatischen C₁-C₁₅ Alkoxy-Gruppen, unsubstituierten C₅-C₂₄ Aryl-Gruppen, substituierten C₅-C₂₄ Aryl-Gruppen, unsubstituierten C₅-C₂₄ Heteroaryl-Gruppen, substituierten C₅-C₂₄ Heteroaryl-Gruppen, unsubstituierten C₅-C₂₄ Aryloxy-Gruppen, substituierten C₅-C₂₄ Aryloxy-Gruppen, unsubstituierten C₅-C₂₄ Heteroaryloxy-Gruppen, substituierten C₅-C₂₄ Heteroaryloxy-Gruppen; X ausgewählt ist aus der Gruppe bestehend aus Halogenatomen; Y ausgewählt ist aus der Gruppe bestehend aus CH₂, O, S, SO, SO₂ und NR⁴, wobei R⁴ ausgewählt ist aus der Gruppe bestehend aus Wasserstoff, unsubstituierten linearen oder verzweigten monovalenten gesättigten aliphatischen C₁-C₁₅ Kohlenwasserstoff-Gruppen, substituierten linearen oder verzweigten monovalenten gesättigten aliphatischen C₁-C₁₅ Kohlenwasserstoff-Gruppen und Cycloalkyl-Gruppen; und Z ausgewählt ist aus der Gruppe bestehend aus Sauerstoff und zwei Wasserstoffatomen; wobei die Zusammensetzung fähig ist das Wachstum oder Überleben eines Mikroorganismus, welcher besagte Infektion verursacht, zu hemmen.

2. Die Zusammensetzung zur Verwendung gemäß Anspruch 1, wobei R¹ ausgewählt ist aus der Gruppe bestehend aus Wasserstoff, substituierten und unsubstituierten C₁-C₅ Alkyl-Gruppen, C₃-C₁₀ Cycloalkyl-Gruppen, substituierten und unsubstituierten C₆-C₁₄ Aryl-Gruppen, substituierten und unsubstituierten C₅-C₁₄ Heteroaryl-Gruppen und Gruppen welche in lebenden Körpern hydrolisiert werden können; R² ausgewählt ist aus der Gruppe bestehend aus unsubstituierten Amino-Gruppen und Amino-Gruppen, die mit einer oder zwei C₁-C₅ Alkyl-Gruppen substituiert sind; R³ ausgewählt ist aus der Gruppe bestehend aus Wasserstoff, substituierten und unsubstituierten C₁-C₅ Alkyl-Gruppen, C₃-C₁₀ Cycloalkyl-Gruppen, substituierten und unsubstituierten C₁-C₅ Alkoxy-Gruppen, substituierten und unsubstituierten C₅-C₁₄ Aryl-Gruppen, substituierten und unsubstituierten C₅-C₁₄ Heteroaryl-Gruppen und substituierten und unsubstituierten C₅-C₁₄ Aryloxy-Gruppen; und X ausgewählt ist aus der Gruppe bestehend aus Cl, F und Br.

3. Die Zusammensetzung zur Verwendung gemäß Anspruch 1, wobei R¹ ausgewählt ist aus der Gruppe bestehend aus Wasserstoff, substituierten und unsubstituierten C₁-C₅ Alkyl-Gruppen und Gruppen welche in lebenden Körpern hydrolisiert werden können; R² ausgewählt ist aus der Gruppe bestehend aus unsubstituierten Amino-Gruppen und Amino-Gruppen, die mit einer oder zwei C₁-C₅ Alkyl-Gruppen substituiert sind; R³ ausgewählt ist aus der Gruppe bestehend aus C₃-C₁₀ Cycloalkyl-Gruppen; X ausgewählt ist aus der Gruppe bestehend aus Cl und F; Y CH₂ umfasst; und Z zwei Wasserstoffatome umfasst.

4. Die Zusammensetzung zur Verwendung gemäß Anspruch 1, wobei das Fluoroquinolon in einer Menge von 0.0001 bis 10 Gew.-% vorhanden ist.

5. Die Zusammensetzung zur Verwendung gemäß Anspruch 4, wobei das Fluoroquinolon in einer Menge von 0.01 bis 5 Gew.-% vorhanden ist.

6. Die Zusammensetzung zur Verwendung gemäß Anspruch 5, wobei das Fluoroquinolon in einer Menge von 0.01 bis 1 Gew.-% vorhanden ist.

7. Die Zusammensetzung zur Verwendung gemäß Anspruch 5, weiterhin einen entzündungshemmenden Wirkstoff umfassend.

8. Die Zusammensetzung zur Verwendung gemäß Anspruch 7, wobei besagter entzündungshemmender Wirkstoff ausgewählt ist aus der Gruppe bestehend aus Glucocorticosteroiden, nicht-steroidalen entzündungshemmenden Wirkstoffen, Inhibitoren der Cytokin-Produktion und Mischungen daraus.

9. Die Zusammensetzung zur Verwendung gemäß Anspruch 4, weiterhin umfassend ein Material ausgewählt aus der Gruppe bestehend aus Puffern, Tensiden, Viskositätsmodifikatoren, Tonizität-einstellenden Mitteln, Konservierungsmitteln, Antioxidantien, zusätzlichen Medikamenten und Kombinationen daraus.

10. Die Zusammensetzung zur Verwendung gemäß Anspruch 8, weiterhin umfassend ein Material ausgewählt aus der Gruppe bestehend aus Puffern, Tensiden, Viskositätsmodifikatoren, Tonizität-einstellenden Mitteln, Konservierungsmitteln, Antioxidantien, zusätzlichen Medikamenten und Kombinationen daraus.

11. Die Zusammensetzung zur Verwendung gemäß Anspruch 4, wobei das Fluoroquinolon die Formel III besitzt

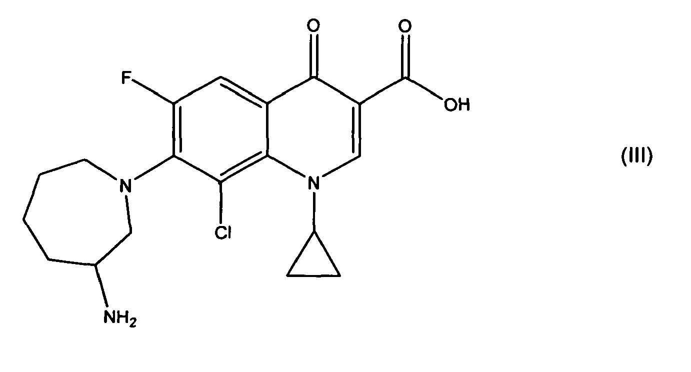

12. Die Zusammensetzung zur Verwendung gemäß Anspruch 7, wobei das Fluoroquinolon die Formel III besitzt

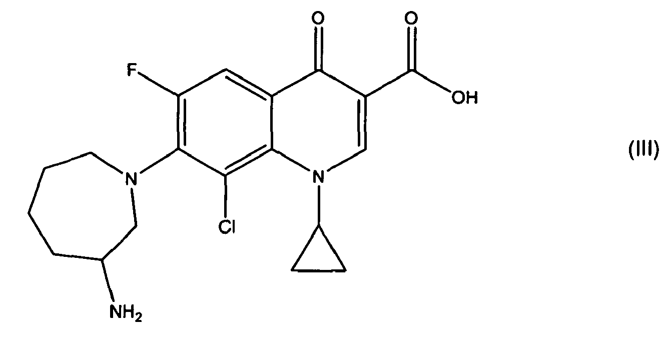

13. Die Zusammensetzung zur Verwendung gemäß Anspruch 1, umfassend ein einziges Enantiomer des Fluoroquinolons, welches die Formel I besitzt.

14. Die Zusammensetzung zur Verwendung gemäß Anspruch 11, umfassend ein einziges Enantiomer des Fluoroquinolons, welches die Formel III besitzt.

15. Die Zusammensetzung zur Verwendung gemäß Anspruch 12, umfassend ein einziges Enantiomer des Fluoroquinolons, welches die Formel III besitzt.

16. Die Zusammensetzung zur Verwendung gemäß Anspruch 4, wobei die Zusammensetzung in Form einer Lösung, Creme, Emulsion, Suspension, Salbe oder eines Gels vorliegt.

17. Die Zusammensetzung zur Verwendung gemäß Anspruch 1, wobei die Zusammensetzung für die topische, orale oder systemische Anwendung ist.

18. Die Zusammensetzung zur Verwendung gemäß Anspruch 17, wobei die Zusammensetzung für die topische Anwendung ist.

19. Die Zusammensetzung zur Verwendung gemäß Anspruch 18, wobei die Infektion Otitis Extema, Otitis Media oder eine Kombination daraus ist.

20. Die Zusammensetzung zur Verwendung gemäß Anspruch 18, wobei die Infektion ausgewählt ist aus der Gruppe bestehend aus Sinusitis, Nasopharyngitis, Oropharyngitis und Kombinationen daraus.

21. Die Zusammensetzung zur Verwendung gemäß Anspruch 1, wobei das Fluoroquinolon die Formel III besitzt.

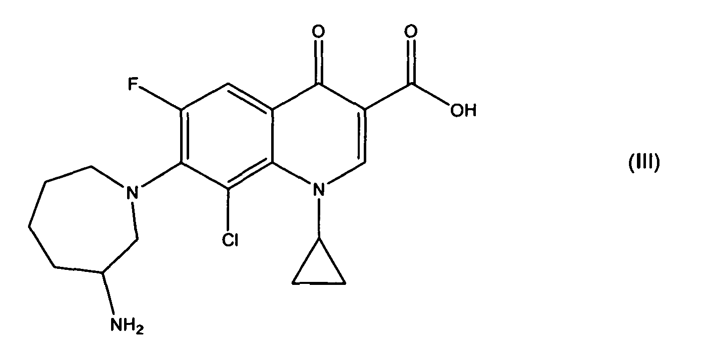

22. Die Zusammensetzung zur Verwendung gemäß Anspruch 21, wobei die Zusammensetzung für die topische, orale oder systemische Anwendung ist.

23. Die Zusamemensetzung zur Verwendung gemäß Anspruch 22, wobei die Zusammensetzung für die topische Anwendung ist.

24. Die Zusammensetzung zur Verwendung gemäß Anspruch 23, wobei die Infektion Otitis Externa, Otitis Media oder eine Kombination daraus ist.

25. Die Zusammensetzung zur Verwendung gemäß Anspruch 23, wobei die Infektion ausgewählt ist aus der Gruppe bestehend aus Sinusitis, Nasopharyngitis, Oropharyngitis und Kombinationen daraus.

26. Die Zusammensetzung zur Verwendung gemäß Anspruch 1, wobei besagter Mikroorganismus ein Bakterium ist, welches gegenüber einem Antibiotikum resistent ist.

27. Die Zusammensetzung zur Verwendung gemäß Anspruch 21, wobei die Zusammensetzung fähig ist, das Wachstum oder Überleben eines Mikroorganismus, welcher gegenüber einem Antibiotikum resistent ist, zu hemmen.

## Revendications

1. Composition destinée au traitement, à l'atténuation, à l'amélioration, ou à la prévention d'une infection d'une oreille ou d'une partie d'une voie respiratoire supérieure d'un sujet, la composition comprenant une fluoroquinolone de formule I ou des sels de celle-ci, la fluoroquinolone étant présente en une quantité efficace pour traiter, atténuer, améliorer, ou prévenir ladite infection, 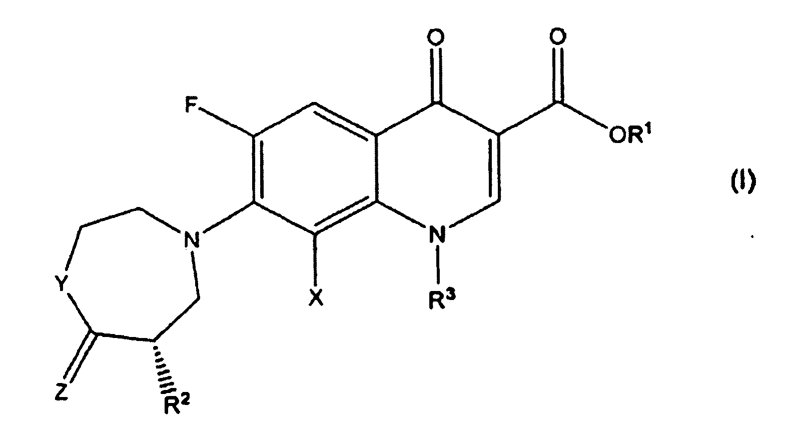 dans laquelle R¹ est choisi dans le groupe constitué par un atome d'hydrogène, des groupes hydrocarbure aliphatiques monovalents, saturés, non substitués, à chaîne linéaire ou ramifiée en C₁-C₁₅, des groupes hydrocarbure aliphatiques monovalents, saturés, substitués, à chaîne linéaire ou ramifiée en C₁-C₁₅, des groupes cycloalkyle, des groupes aryle non substitués en C₅-C₂₄, des groupes aryle substitués en C₅-C₂₄, des groupes hétéroaryle non substitués en C₅-C₂₄, des groupes hétéroaryle substitués en C₅-C₂₄, et des groupes qui peuvent être hydrolysés dans des corps vivants ; R² est choisi dans le groupe constitué par un atome d'hydrogène, un groupe amino non substitué, et des groupes amino substitués par un ou deux groupes hydrocarbure aliphatiques monovalents, saturés, substitués ou non substitués, à chaîne linéaire ou ramifiée, en C₁-C₁₅ ; R³ est choisi dans le groupe constitué par un atome d'hydrogène, des groupes hydrocarbure aliphatiques monovalents, saturés, non substitués, à chaîne linéaire ou ramifiée en C₁-C₁₅, des groupes hydrocarbure aliphatiques monovalents, saturés, substitués, à chaîne linéaire ou ramifiée en C₁-C₁₅, des groupes cycloalkyle, des groupes alcoxy aliphatiques monovalents, saturés, non substitués, à chaîne linéaire ou ramifiée en C₁-C₁₅, des groupes alcoxy aliphatiques monovalents, saturés, substitués, à chaîne linéaire ou ramifiée en C₁-C₁₅, des groupes aryle non substitués en C₅-C₂₄, des groupes aryle substitués en C₅-C₂₄, des groupes hétéroaryle non substitués en C₅-C₂₄, des groupes hétéroaryle substitués en C₅-C₂₄, des groupes aryloxy non substitués en C₅-C₂₄, des groupes aryloxy substitués en C₅-C₂₄, des groupes hétéroaryloxy non substitués en C₅-C₂₄, et des groupes hétéroaryloxy substitués en C₅-C₂₄ ; X est choisi dans le groupe constitué par des atomes d'halogène ; Y est choisi dans le groupe constitué par CH₂, O, S, SO, SO₂, et NR⁴, R⁴ étant choisi dans le groupe constitué par un atome d'hydrogène, des groupes hydrocarbure aliphatiques monovalents, saturés, non substitués, à chaîne linéaire ou ramifiée en C₁-C₁₅, des groupes hydrocarbure aliphatiques monovalents, saturés, substitués, à chaîne linéaire ou ramifiée en C₁-C₁₅, et des groupes cycloalkyle ; et Z est choisi dans le groupe constitué par un atome d'oxygène et deux atomes d'hydrogène ; et la composition étant capable d'inhiber une croissance ou une survie d'un microorganisme responsable de ladite infection.

2. Composition destinée à être utilisée selon la revendication 1, R¹ étant choisi dans le groupe constitué par un atome d'hydrogène, des groupes alkyle substitués ou non substitués en C₁-C₅, des groupes cycloalkyle en C₃-C₁₀, des groupes aryle substitués ou non substitués en C₆-C₁₄, des groupes hétéroaryle substitués ou non substitués en C₅-C₁₄, et des groupes qui peuvent être hydrolysés dans des corps vivants ; R² est choisi dans le groupe constitué par un groupe amino non substitué et des groupes amino substitués par un ou deux groupes alkyle en C₁-C₅ ; R³ est choisi dans le groupe constitué par un atome d'hydrogène, des groupes alkyle substitués ou non substitués en C₁-C₅, des groupes cycloalkyle en C₃-C₁₀, des groupes alcoxy substitués ou non substitués en C₁-C₅, des groupes aryle substitués ou non substitués en C₅-C₁₄, des groupes hétéroaryle substitués ou non substitués en C₅-C₁₄, et des groupes aryloxy substitués ou non substitués en C₅-C₁₄, et X est choisi dans le groupe constitué par Cl F, et Br.

3. Composition destinée à être utilisée selon la revendication 1, R¹ étant choisi dans le groupe constitué par un atome d'hydrogène, des groupes alkyle substitués ou non substitués en C₁-C₅ et des groupes qui peuvent être hydrolysés dans des corps vivants ; R² étant choisi dans le groupe constitué par un groupe amino non substitué et des groupes amino substitués par un ou deux groupes alkyle en C₁-C₅ ; R³ est choisi dans le groupe constitué par les groupes cycloalkyle en C₃-C_{10;} X est choisi dans le groupe constitué par Cl et F ; Y comprend CH₂ ; et Z comprend deux atomes d'hydrogène.

4. Composition destinée à être utilisée selon la revendication 1, la fluoroquinolone étant présente en une quantité de 0,0001 à 10 % en poids.

5. Composition destinée à être utilisée selon la revendication 4, la fluoroquinolone étant présente en une quantité de 0,01 à 5 % en poids.

6. Composition destinée à être utilisée selon la revendication 5, la fluoroquinolone étant présente en une quantité de 0,01 à 1 % en poids.

7. Composition destinée à être utilisée selon la revendication 5, comprenant en outre un agent anti-inflammatoire.

8. Composition destinée à être utilisée selon la revendication 7, ledit agent anti-inflammatoire étant choisi dans le groupe constitué par les glucocorticoïdes, les anti-inflammatoires non stéroïdiens, les inhibiteurs de la production de cytokines, et leurs mélanges.

9. Composition destinée à être utilisée selon la revendication 4, comprenant en outre un matériau choisi dans le groupe constitué par les tampons, les surfactants, les modificateurs de la viscosité, les agents d'ajustement de la tonicité, les conservateurs, les antioxydants, des médicaments supplémentaires, et des combinaisons de ceux-ci.

10. Composition destinée à être utilisée selon la revendication 8, comprenant en outre un matériau choisi dans le groupe constitué par les tampons, les surfactants, les modificateurs de la viscosité, les agents d'ajustement de la tonicité, les conservateurs, les antioxydants, des médicaments supplémentaires, et des combinaisons de ceux-ci.

11. Composition destinée à être utilisée selon la revendication 4, la fluoroquinolone répondant à la formule III.

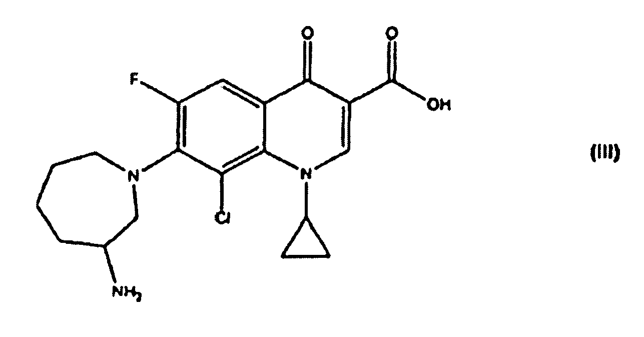

12. Composition destinée à être utilisée selon la revendication 7, la fluoroquinolone répondant à la formule III.

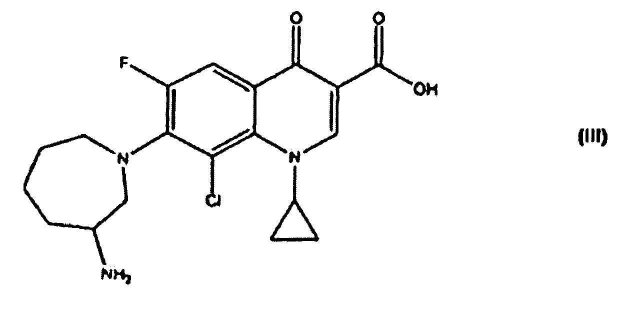

13. Composition destinée à être utilisée selon la revendication 1, comprenant un énantiomère unique de la fluoroquinolone de formule I.

14. Composition destinée à être utilisée selon la revendication 11, comprenant un énantiomère unique de la fluoroquinolone de formule III.

15. Composition destinée à être utilisée selon la revendication 12, comprenant un énantiomère unique de la fluoroquinolone de formule III.

16. Composition destinée à être utilisée selon la revendication 4, la composition se trouvant sous la forme d'une solution, d'une crème, d'une émulsion, d'une suspension, d'un onguent, ou d'un gel.

17. Composition destinée à être utilisée selon la revendication 1, la composition étant destinée à une administration topique, orale, ou systémique.

18. Composition destinée à être utilisée selon la revendication 17, la composition étant destinée à une administration topique.

19. Composition destinée à être utilisée selon la revendication 18, l'infection étant une otite externe, une otite moyenne, ou une combinaison de celles-ci.

20. Composition destinée à être utilisée selon la revendication 18, l'infection étant choisie dans le groupe constitué par la sinusite, une rhino-pharyngite, une oropharyngite, et des combinaisons de celles-ci.

21. Composition destinée à être utilisée selon la revendication 1, la fluoroquinolone répondant à la formule III.

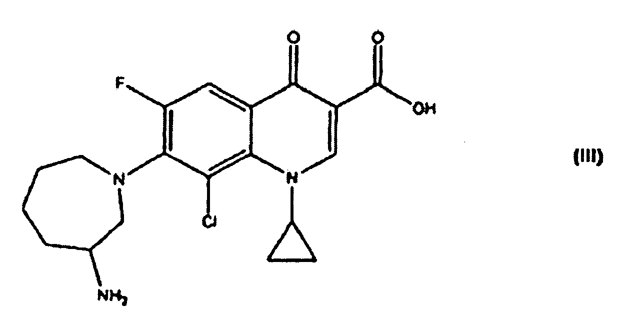

22. Composition destinée à être utilisée selon la revendication 21, la composition étant destinée à une administration topique, orale, ou systémique.

23. Composition destinée à être utilisée selon la revendication 22, la composition étant destinée à une administration topique.

24. Composition destinée à être utilisée selon la revendication 23, l'infection étant une otite externe, une otite moyenne, ou une combinaison de celles-ci.

25. Composition destinée à être utilisée selon la revendication 23, l'infection étant choisie dans le groupe constitué par la sinusite, une rhino-pharyngite, une oro-pharyngite, et des combinaisons de celles-ci.

26. Composition destinée à être utilisée selon la revendication 1, ledit microorganisme étant une bactérie qui est résistante à un antibiotique.

27. Composition destinée à être utilisée selon la revendication 21, la composition étant capable d'inhiber une croissance ou une survie d'un microorganisme résistant à un antibiotique.
